# EUROPEAN PATENT APPLICATION

(11) **EP 4 059 421 A1**
(43) Date of publication of application: **21.09.2022**
(21) Application number: 21163642.8
(22) Date of filing: 19.03.2021
(51) Int. Cl.: A61B 5/12, A61B 5/11, G10K 15/04

(54) **SYSTEM FOR ASSESSING PROPERTIES OF A SUBJECT'S SENSE OF HEARING**

(71) Applicant: Koninklijke Philips N.V., 5656 AG Eindhoven (NL)
(72) Inventor: WRIGHT, Christopher John, 5656 AE Eindhoven (NL); LAWRENSON, Matthew John, 5656 AE Eindhoven (NL)
(74) Representative: Philips Intellectual Property & Standards

(57) **Abstract**

A system (10) is provided for assessing properties of a subject's hearing. The system includes means (12) for generating a 3D sound distribution comprising one or more sound stimuli with defined real or virtual source locations (24) in 3D space (22). The source locations can be controlled to be at defined positions relative to the head of a subject to create a test-specific acoustic sound-set. The subject is prompted with instructions to perform particular movements of their head dependent upon their perception of the sound, for example their perception of properties of the sound such as loudness, sound source location, or relative properties of two sound stimuli. The position of the subject's head, or certain features thereof, after following the instructions is measured using a position sensing apparatus (14), and used as a metric to compute quantitative parameters of the subject's hearing.

## Description

### FIELD OF THE INVENTION

The present invention relates to a system for assessing properties of a subject's sense of hearing.

### BACKGROUND OF THE INVENTION

The standard method for testing a person's hearing is pure-tone audiometry. It can be used to identify hearing threshold levels of an individual, enabling degree and type of hearing loss to be determined. It is subjective in character since it relies on a patient's verbal response to a pure tone stimuli. The test is in general vulnerable to two levels of subjectivity and error, originating from the doctor's and the patient's reporting and judgement.

It is also limited in the hearing properties it can assess. It can only measure audibility thresholds, and cannot measure other aspects of hearing such as sound localization and left/right ear sensitivity imbalance. It is also unable to give nuance or detail in its testing of hearing thresholds since it calls for just a binary response from the patient (whether they can or cannot hear a sound stimulus).

An improved system for hearing assessment would therefore be of value.

### SUMMARY OF THE INVENTION

The invention is defined by the claims.

According to examples in accordance with an aspect of the invention, there is provided a system for use in assessing hearing of a subject, the system comprising:
an acoustic apparatus operable to generate a sound distribution in a 3D space, the sound distribution comprising one or more sound stimuli having controllable real or virtual sound source locations in the 3D space;
a position sensing apparatus for use in detecting a position or pose of at least one or more anatomical features of a subject's head in the 3D space during a testing routine;
a processing arrangement configured to perform a testing routine, the testing routine comprising:
   generating a sound distribution in a 3D space comprising one or more sound stimuli having defined sound source locations in the 3D space, using the acoustic apparatus;
   generating instructions for communication to the subject during generation of the sound distribution, the instructions for prompting the subject to respond to the sound stimuli with movements of their head based on their perception of the sound;
   determining a position or pose in the 3D space of at least one or more anatomical features of the subject's head after generation of the instructions, based on data from the position sensing apparatus; and
   determining one or more quantitative properties of the subject's sense of hearing based on a position or pose of the at least one or more anatomical features of the subject's head in the 3D space after issuance of the instructions.

Assessment may be based on a position of mid-point between two or more anatomical features of the subject's head in some examples.

Embodiments of the invention are based on providing an objective assessment of a subject's hearing by delivering one or multiple audio stimuli in a 3D configuration around the subject's head, and issuing instructions prompting the subject to respond with head movements based on how they perceive the sound. In particular, the subject may be prompted to move their head to a location at which the sound they are perceiving exhibits or matches a pre-defined auditory quality or property, e.g. relating to loudness of a single sound stimulus or relative loudness of two stimuli. Various other examples also exist as will be explained later. A position sensing means is used to sense a position or pose of the subject's head, or features thereof, after issuance of the instructions and a measure of one or more properties of the subject's hearing are quantified based on the positioning of their head or head feature(s) in the 3D space relative to the produced audible stimuli. This enables an objective, quantitative measure to be obtained of various properties of the subject's sense of hearing.

Determining the one or more quantitative properties of the subject's sense of hearing may be further based on the source locations of one or more of the generated sound stimuli.

For example, the determining the one or more quantitative properties of the subject's sense of hearing may be based on a position or pose of the at least one or more anatomical features of the subject's head in the 3D space relative to the sound stimuli source locations. The processing arrangement may calculate a deviation from the positions of the one or more sound stimuli source locations.

The instructions may be for prompting the subject to move their head to a location at which the perceived sound matches a defined (target) acoustic quality. The defined quality means a particular acoustic property of the perceived sound, e.g. a maximal or minimal volume or a balance in volume of two sources, and/or a certain perceived direction of origin or location of origin of the sound source, and/or a perception of the sound being at a particular target location relative to the subject (e.g. directly above their head, and/or at a mid-point between their ears).

The processing arrangement may further be configured to perform a set-up or calibration phase, before the testing routine.

For example, the processing arrangement may be further configured, in advance of generating the instructions, to determine an initial position or pose of at least one or more anatomical features of the subject's head in the 3D space; and configure the sound stimuli source locations in the 3D space based on said initial position or pose of the at least one or more anatomical features of the subject's head.

The configuring the sound stimuli source locations may comprise: controlling the source locations to be at defined positions relative to the determined initial position(s) of the at least one or more anatomical features of the subject's head; and wherein the locations of the sound sources in the 3D space remain fixed relative to a reference frame of the 3D space after the configuration, independent of subsequent movement of the subject's head.

Here, an initial set-up procedure comprises determining the source locations at which the sound stimuli should be created, based on the initial position of the subject's head, or features therefore, and the sound stimuli are then generated having those source locations. Each source location may be controlled to be at a defined spatial displacement from one or more of the anatomical features of the subject's head, or at a midpoint between two or more features for example. This can generate a 3D array of sound stimuli at particular locations relative to the subject's head.

After the sound source locations have been determined and set, they are then preferably anchored to their locations in 3D space (i.e. in the reference frame or co-ordinate system of the 3D space), regardless of subsequent movements of the subject's head, i.e. they are not held at a fixed displacement relative to a reference frame of the subject's head.

This procedure allows for controlled implementation of any of a wide variety of hearing examinations, wherein the required sound stimulus configuration can be dynamically controlled, and can be adjusted to the sitting position of the subject.

In some embodiments, the system may further comprise a datastore storing configuration instructions for each of a set of different testing routines, each testing routine for assessing a different set of one or more properties of the subject's sense of hearing, wherein the configuration instructions for each testing routine define:
a set of one or more acoustic stimuli to be generated (i.e. acoustic properties of the stimuli); and
spatial locations relative to at least one or more anatomical features of the subject's head, or relative to a midpoint between two or more anatomical features, at which the real or virtual sources of the sounds should be generated.

In other words, the initial source locations of the sound stimuli may be defined in the dataset relative to the position of the subject's head, or features thereof. For testing different hearing ability properties, different sound configurations may be necessary.

The same or a different dataset may also store for each of the set of different testing routines the instructions which should be issued to the subject during the assessment.

The same or a different dataset may also store for each of the set of different testing routines computation algorithms or functions for use in determining the relevant quantitative properties of the subject's hearing based on acquired position data of the at least one or more features of the subject's head after issuance of the instructions, e.g. it defines a metric to use for calculating the quantitative properties of the hearing.

In accordance with one or more embodiments, the position sensing apparatus may comprise: at least one imaging device; and/or a LIDAR sensing apparatus. These represent different examples of spatial sensing devices. The position sensing apparatus may comprise a combination of a spatial sensing device (such as those listed) and a processing arrangement configured to process acquired sensing data to derive position information.

In accordance with one or more embodiments, determining a position in 3D space of at least one or more anatomical features of the subject's head may comprise determining a position in 3D space of at least one of the subject's ears.

A position and/or orientation (pose) of one or both ears can be identified. In some examples, a position and/or orientation of an entrance to the ear canal may be determined for example. A mid-point between the ears may be identified in some examples.

In accordance with one or more embodiments, the acoustic apparatus may comprise: a laser-based sound generation apparatus; an ultrasound apparatus; and/or one or more speakers.

In accordance with one or more embodiments, the determining the quantitative properties of the subject's hearing may comprise determining one or more of: a left/right ear sensitivity imbalance; an ear sensitivity magnitude; an ear directional sensitivity.

In accordance with one or more embodiments, the determining the quantitative properties of the subject's hearing may comprise calculating, based on the data from the position sensing apparatus: a displacement of at least one or more anatomical features of the subject's head, or a mid-point therebetween, from a source location in the 3D space of at least one of the sound stimuli; and/or a pose of the subject's head in the 3D space.

In one set of examples, this may comprise determining a deviation of a midpoint between a subject's ears along a horizontal axis from the horizontal position of a generated sound stimulus source. If the subject is prompted to move to where the sound is perceived to be directly above their head or mid-way between their ears, this deviation provides a measure of left/right ear sensitivity imbalance. This is something a standard pure-tone audiometry test cannot measure.

In general, a pose of the subject's head may be characterized by one or more different spatial characteristics. One option is a facing direction of the head, defined for example as a vector direction of an axis running from the front to the back of head, relative to a co-ordinate system of the 3D space. Additionally or instead, another option may be a tilt of the subject's head, e.g. a direction vector of an axis running from the bottom to the top of head relative to a co-ordinate system of the 3D space. Measuring pose may be done when directional sensitivity properties are being assessed.

The system may further comprise a sensory output device for generating a sensory output indicative of the generated instructions.

Examples in accordance with a further aspect of the invention provide a computer-implemented method for testing hearing of a subject, the method comprising performing a testing routine. The testing routine comprises generating a sound distribution in a 3D space, the sound distribution comprising one or more sound stimuli having controllable real or virtual sound source locations in the 3D space. The testing further routine comprises generating instructions for communication to the subject during generation of the sound distribution, the instructions for prompting the subject to respond to the sound stimuli by performing movements of their head based on their perception of the sound. The testing routine further comprises determining a position in the 3D space of at least one or more anatomical features of the subject's head after generation of the instructions. The testing further routine comprises determining one or more quantitative properties of the subject's sense of hearing based on a position or pose of the at least one or more anatomical features of the subject's head in the 3D space after issuance of the instructions.

In accordance with one or more embodiments, the instructions may be for prompting the subject to move their head to a location at which the perceived sound exhibits a defined target acoustic property.

The method may further comprise, in advance of generating the instructions: determining an initial position or pose of at least one or more anatomical features of the subject's head in the 3D space; and configuring the sound stimuli source locations based on said initial position or pose of the at least one or more anatomical features of the subject's head.

Examples in accordance with a further aspect of the invention provide a computer program product comprising computer program code, the computer program code being executable on a processing arrangement. When the processing arrangement is operatively coupled with an acoustic apparatus operable to generate a configurable sound distribution in space, and with a position sensing apparatus, the code is configured to cause the processing arrangement to perform a method in accordance with any example or embodiment outlined above or described below, or in accordance with any claim of this application.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying schematic drawings, in which:
Fig. 1 outlines components of an example system according to one or more embodiments;
Fig. 2 illustrates operation of the system to perform a hearing test;
Fig. 3 shows an example system according to one or more embodiments;
Fig. 4 outlines an example processing workflow of an example system; and
Figs. 5-8 illustrate implementation of an example test routine in which left/right ear sensitivity imbalance is measured based on measuring horizontal displacement of a subject's head relative to a generated sound source after issuance of instructions.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system for assessing properties of a subject's hearing. The system includes means for generating a 3D sound distribution comprising one or more sound stimuli with defined real or virtual source locations in space. The source locations can be controlled to be at defined positions relative to the head of a subject to create a test-specific acoustic sound-set. The subject is prompted with instructions to perform particular movements of their head dependent upon their perception of the sound, for example their perception of properties of the sound such as loudness, sound source location, or relative properties of two sound stimuli. The position of the subject's head, or certain features thereof, is measured using a position sensing apparatus, and used as a metric to compute quantitative parameters of the subject's hearing.

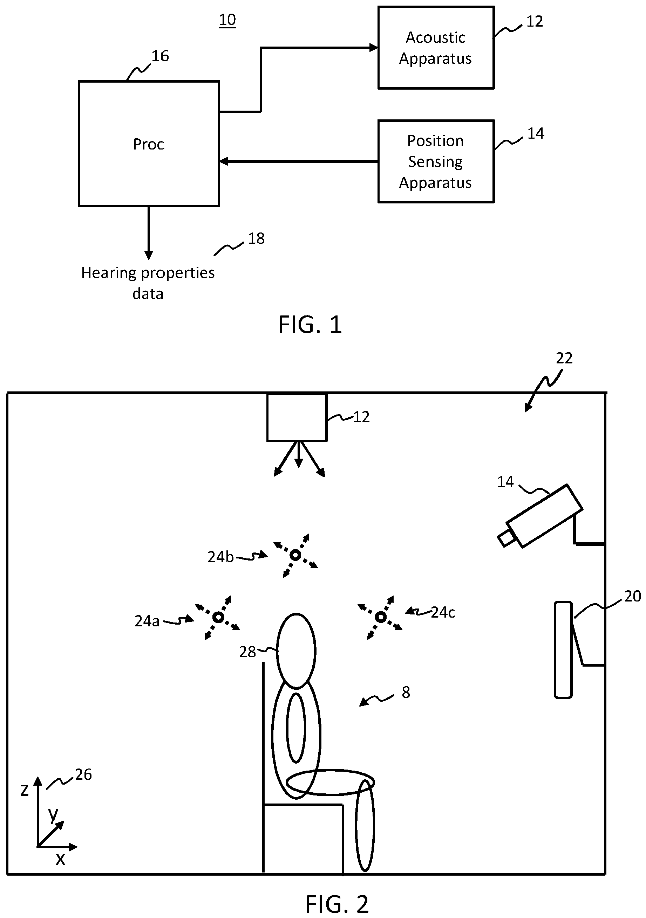

Fig. 1 outlines the architecture of an example system 10 for use in assessing hearing of a subject according to one or more embodiments.

The system 10 comprises an acoustic apparatus 12 operable to generate a sound distribution in a 3D space, the sound distribution comprising one or more sound stimuli having controllable real or virtual sound source locations in the 3D space.

The system 10 further comprises a position sensing apparatus 14 for use in detecting a position or pose of at least one or more anatomical features of a subject's head in the 3D space during a testing routine.

The system 10 further comprises a processing arrangement 16, operatively coupled with the acoustic apparatus 12 and the position sensing apparatus 14, and configured to implement a testing routine.

Fig. 2 schematically illustrates the system 10 in use, during implementation of a testing routine. The processing arrangement 10 is not shown in Fig. 2, but in practice would be operatively coupled to each of the acoustic apparatus 12 and the position sensing apparatus 14. The test routine is performed within a 3D test space 22 (herein "3D space"). The 3D space is associated with a co-ordinate system 26 fixed relative to a reference frame of the 3D space. A subject 8 is located in the 3D space 22 during the test routine. Performance of the test routine comprises the processing arrangement 16 performing the following steps.

The processing arrangement controls generation of a sound distribution in a 3D space 22 comprising one or more sound stimuli having defined sound source locations 24a 24b, 24c in the 3D space, using the acoustic apparatus 12.

Instructions are generated for communication to the subject 8 during generation of the sound distribution, the instructions for prompting the subject to respond to the sound stimuli 24 with movements of their head 28 based on their perception of the sound. The processing arrangement 16 may be arranged to transmit the generated instructions to a sensory output device 20 to generate a sensory output representative of the instructions. The sensory output device may include for example a display unit with a display screen which displays visual instructions, and/or a sound generator which delivers the instructions acoustically (in verbal form). The sensory output device 20 may be comprised as a part of the system of the claimed invention, or it may be an auxiliary device with which the system is merely arranged to communicate.

The processing arrangement 16 is further configured to determine a position or pose in the 3D space 22 of at least one or more anatomical features of the subject's head 28 after generation of the instructions, based on data from the position sensing apparatus 14.

The processing arrangement 16 is further configured to determine one or more quantitative properties of the subject's sense of hearing based on a position or pose of the at least one or more anatomical features of the subject's head 28 in the 3D space 22 after issuance of the instructions.

With regards to the position sensing apparatus 14, this is arranged to capture data indicative of position and/or pose of the subject's head or one or more anatomical features thereof (e.g. the ears). The position and/or pose information is preferably 3D position and/or pose information, but, depending upon the requirements of the testing routine, 2D sensing data may be sufficient in some cases.

The position sensing apparatus 14 may in general comprise two components: a (spatial) sensing component, e.g. one or more cameras or a LIDAR system, and a processing module for processing the raw spatial sensing data to derive usable position sensing information of the subject which can be used in the main processing flow of the test routine. However, in an alternative arrangement, the function of the processing module may be performed by the main processing arrangement 16, for example by a particular module of the processing arrangement 16.

With regards to the spatial sensing part of the position sensing apparatus 14, this may comprise for example one or more cameras. The cameras may be standard 2D cameras, and for example a plurality of cameras can be arranged facing in different directions toward the test space, enabling positioning of features of the subject's head in three-dimensional co-ordinates to be determined. It is also possible to use just a single RGB camera system. One example is described for instance in the paper: Mehta, Dushyant et al, VNect: Real-time 3D Human Pose Estimation with a Single RGB Camera, ACM Transactions on Graphics, (2017).

According to further examples, one or more RGBD cameras may be used. An RGBD camera typically generates standard RGB images in combination with a corresponding depth image, where the depth image is an image channel in which each pixel value relates to a distance between the image plane and a corresponding object in the RGB image. Thus an RGBD camera can acquire 3D image data with the use of only one camera unit. The "Kinect" camera is an example of an RGBD camera.

Another type of imaging device could instead be used such as for example a thermal imaging device.

Instead of an image capturing device such as a camera, a different form of spatial sensing or spatial mapping device can be used, such as for example a LIDAR system. LIDAR is a method for measuring distances based on illuminating a target object with laser light and measuring the reflection with a sensor. The laser return time and wavelength can be used to determine distance, and over the whole of an object or scene, can be used to form a digital 3D representation of the object or scene.

Any other spatial sensing means also be considered, including, for example, arrangements making use of tracking markers or responders mounted at locations on the subject's head or head features, whose position can be tracked visually or using transfer of electromagnetic or acoustic signals from a transmitter to and/or from the markers.

With regards to the processing part of the position sensing apparatus 14, this can be implemented with one or more image processing or machine vision algorithms configured to detect within acquired spatial (e.g. image) data one or more anatomical features of the subject or the subject's head. By way of non-limiting example, one or more image processing algorithms may be configured to detect one or more of:
a location of the ear canal entrance, or associated structures, defined by a single spatial point in the 3D space;
a location of one or more points on the subject's ears, e.g. a tip of the ear of a tip of the ear lobe,
an orientation or pose of the ear or ear canal;
subject earlobe shape or geometry information;
a location of a tip of the subject's head and/or a location of a bottom-most point of a subject's chin;
a pose or orientation (e.g. axial rotation and tilt) of a subject's head.

Pose or orientation information may be defined by a 2D or 3D vector in a co-ordinate system of the 3D space, and position information by a single co-ordinate point in the 3D space.

The algorithms may for example employ shape matching algorithms for identifying certain features, and/or may employ image segmentation techniques to identify outlines of anatomical features, e.g. anatomical model-based image segmentation. In some examples, one or more machine learning algorithms may be employed which have been trained to recognize locations of a certain set of subject anatomical features in a 3D space, based on input spatial sensing (e.g. image) data representative of a region containing one or more of these features, and in a same spatial sensing data format used during the testing routine.

By way of example, the location or orientation of the entrance to the ear canal may be recognized directly from shapes and structures recognizable in the spatial sensing data. Additionally or alternatively, the location and entrance to the ear canal may be inferred from outwardly visible structures, such as the pinna or tip of the ear, where a known or estimated distance and direction of the ear canal entrance from the secondary structures is used to determine the location of the entrance within the acquired spatial sensing data.

In some examples, one set of one or more algorithms may be used to identify the anatomical features or points present in each image or set of spatial data (and e.g. label or outline the features), and another set of one or more algorithms used to determine the location of each identified feature within a co-ordinate system of the 3D space (e.g. a mapping algorithm). The position sensing apparatus may be calibrated before the start of the testing with the 3D space, enabling the position sensing apparatus to provide an output to the processing arrangement 16 comprising a representation of position or orientation of the subject's head or head features in a co-ordinate system of the 3D space.

The system 10 further includes an acoustic apparatus 12 operable to generate an arbitrary sound distribution in the 3D space 22 which comprises one or more sound stimuli having controllable real or virtual sound source locations 24 in the 3D space. There are a range of different acoustic systems which may be used to achieve this propose.

In accordance with one or more embodiments, the acoustic apparatus 12 may be a photoacoustic (laser-based) sound creation system. Such systems use lasers which are swept back and forth across a particular trajectory. The laser radiation interacts with water particles in the air, leading to generation of acoustic emissions. An apparent (virtual) source location of the sound relative to a subject can be created based on control of the sweeping trajectory of the laser. For example, a laser swept along a straight path away and towards a subject's ear canal entrance will generate a sound stimulus with an apparent fixed source location a particular distance from the ear of the subject. The acoustic properties (e.g. amplitude, frequency) of the generated sound stimulus can be precisely controlled through appropriate modulation of the laser beam. For example, the laser is scanned in a plane, or pulsed, at the frequency of the sound which is desired. The system is also able to localize the virtual (apparent) sound stimulus source location very precisely in three dimensions.

One suitable example laser-based photoacoustic system is described in the paper: Ryan M. Sullenberger, Sumanth Kaushik, and Charles M. Wynn, "Photoacoustic communications: delivering audible signals via absorption of light by atmospheric H2O," Opt. Lett. 44, 622-625 (2019).

In accordance with one or more embodiments of the present invention, it is proposed to enhance the functionality of known laser-based sound generators for better localizing sound sources. For example, a swept laser may typically create a sound source having a somewhat diffuse '2D slice' source location in the air. According to one or more embodiments, an adjustment is applied, based on the desired sound source locations, wherein the orientation of the sweeping laser is set such that it always passes through the defined sound source location, and sweeps in-plane with a straight line axis extending directly between the subject's ears. In other words, the laser is swept back and forth, toward and away from the subject's ear. In this way, the sound is perceived as point source as the subject changes their position.

Alternative options for the acoustic apparatus include use of an ultrasonic sound creation system. Here, ultrasonic phased arrays can be used to create interference between ultrasound beams, leading to demodulation of acoustic waves to audible frequencies at a specific points in 3D space. These points in space form the source location of the sound stimuli. The source locations can be controlled based on the interference pattern of the phased array. By way of example, a suitable ultrasonic sound generation apparatus is described in the document EP2920783 B1.

A further option is the use of a fixed speaker system, for example with a plurality of speakers placed around the 3D space 22 or using headphones which are capable of producing perceived sound locations through patterns of audio timing and waveform. In the case of headphones, in order to maintain fixed apparent sound source location relative to the frame of reference of the 3D space 22 (independent of movements of the subject's head), this may require a continuous process of dynamic update of the sound stimuli created by the headphones, based on continuously updated subject head position information.

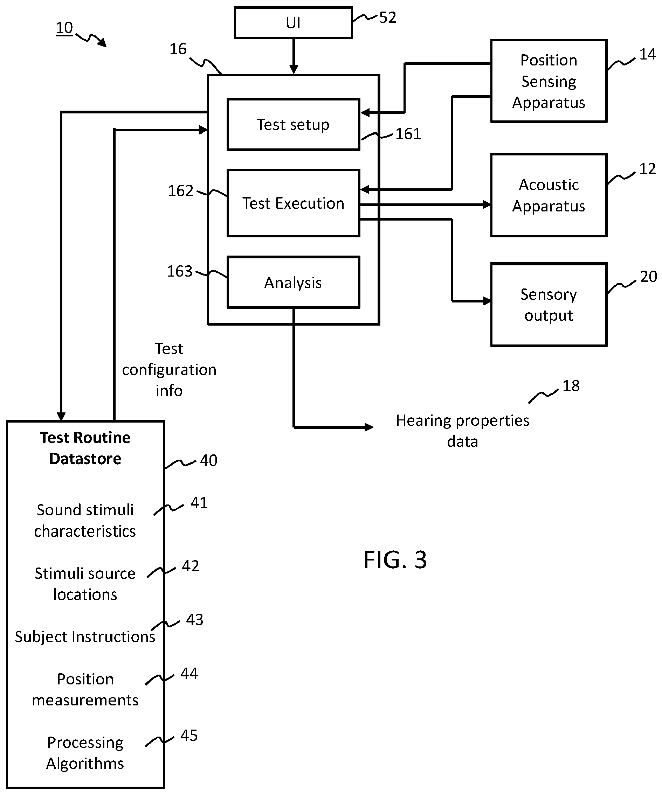

Set-up and implementation of the testing routine according to one or more embodiments of the invention will now be discussed in more detail, with reference to Fig. 3. Fig. 3 outlines components of one example system 10, some or of which may be included in different embodiments.

In use of the system, before implementing a test routine the processing arrangement 16 may be adapted to perform a set-up or calibration routine. This configures the settings or parameters of the system ready for implementing a particular test routine. For example, it comprises determining, based on the test type which is to be performed, the particular sound distribution which needs to be generated by the acoustic apparatus 12, the particular instructions that need to be issued to the subject during the test, what position data needs to be collected after issue of the instructions, and what computation algorithms should be applied to determine the properties of the subject's hearing based on the acquired position data.

For this purpose, the processing arrangement 16 may be adapted to communicate with a local or remote datastore 40 ("Test Routine Datastore") to retrieve configuration instructions for at least one of a set of different testing routines stored in the datastore, each testing routine for assessing a different set of one or more properties of the subject's sense of hearing. Optionally, the processing arrangement may comprise a dedicated test setup module 161 for this purpose. The datastore can be stored on a local memory of the system 10, or can be external to the system and accessible via a further provided communication interface.

The configuration instructions for each testing routine define at least: a set of one or more acoustic stimuli to be generated 41 (i.e. acoustic properties or characteristics of the stimuli); and spatial locations 42 at which the real or virtual sources of the sounds should be generated. For example, the datastore 40 may store a lookup table which records the relevant information for each testing routine. For each testing routine, there may be a single sound source or multiple sound sources to generate. The acoustic properties of each of the sound sources to be generated may include for example volume, pitch, amplitude, frequency, and optionally defined variations of these over time.

Optionally, the same datastore 40 may further define for each testing routine the instructions 43 which should be issued to the subject during the assessment.

The datastore 40 may also define for each testing routine the positioning information 44 which needs to be acquired or derived after issuance of the instructions. For example, it may define which anatomical features of the subject are to be identified, and the type of positioning information to determine, e.g. spatial location, and/or pose (tilt and/or axial rotation).

The datastore 40 may also define one or more computation algorithms or functions 45 for use in determining the relevant quantitative properties of the subject's hearing based on acquired position data of the at least one or more features of the subject's head after issuance of the instructions.

Thus the datastore 40 may store a complete set of implementation information necessary for implementing each of the different testing routines or protocols stored thereon. Alternatively, storage of the different elements 41-45 of the configuration information may distributed among a plurality of different datastores in other examples.

In accordance with one or more embodiments, the system 10 may include a user-interface ("UI") device 52 permitting a user (i.e. an operator of the test) to configure parameters of the testing routine.

The UI 52 may permit a user to directly select a specific testing protocol or routine to be implemented, or the user may select one or more properties of the subject's hearing sense to be tested (e.g. their acoustic positional sensitivity at a certain frequency range and intensity level), and an appropriate test protocol is identified from the datastore 40 by the processing arrangement 16. The processing arrangement may apply one or more test selection algorithms for this purpose. The selection algorithms may include one or more machine learning algorithms for example.

For example, the test routine to be implemented (the 'test identity') may selected by the processing arrangement 16 algorithmically based on input data related to the subject. The input data may include for example: past hearing test data for the subject, demographic information about the subject (e.g. age, sex), relevant medical history information (e.g. a pre-existing hearing condition, or relevant predictive clinical factors). By way of example, a machine learning algorithm may be trained to predict likely hearing defects of a subject based on these predictive data factors. In some examples, at least a subset of the input information provided to the test selection algorithm may comprise, or may be derived from, positioning information provided by the position sensing apparatus. For example, based on relative of position of different facial features of a subject, an estimation may be made as to the age and sex of the subject (e.g. using a further dedicated algorithm), and this information may be provided as an input to the test selection algorithm. Additionally or alternatively, at least a subset of the data may be obtained from an electronic health record (EHR) of the subject. For example the processing arrangement 16 may have a communication module for use in accessing EHR data from a central server via a communication link. Additionally or alternatively, at least a subset of the data may be input to the user interface 52 by the user (operator) of the system. In some examples, a selection algorithm may refine the full set of available testing protocols based on input patient data, and wherein the processing arrangement outputs a representation of the refined list to the UI 52 and a user is prompted to select one of the possible testing routines, for example based on the particular hearing properties of the subject they wish to test.

As discussed, the configuration information for a given test protocol includes locations 42 of the sound sources of each of the sound stimuli to be generated for the test protocol.

In general, it is anticipated that the sound source locations are defined relative to initial positions of at least one or more anatomical features of the subject's head, for example relative to the subject's ear canal entrances, or to a midpoint therebetween. In this way, the pre-defined sound stimulus locations for each test are subject-generic.

To configure generation of the sound distribution for the test, the set-up routine may further include a process of converting or transposing the sound source locations, as defined in the datastore (relative to subject head features), to a set of spatial locations defined within the co-ordinate system 26 of the 3D test space 22. This will then allow the acoustic apparatus to be controlled to generate the sound stimuli at the right locations. This requires obtaining initial positioning information of the subject within the co-ordinate system of the 3D space.

Thus, in advance of the test routine, the processing arrangement 16 may be configured to: determine an initial position or pose of at least one or more anatomical features of the subject's head in the 3D space 22; and configure the sound stimuli source locations 24 in the 3D space based on said initial position or pose of the at least one or more anatomical features of the subject's head.

Configuring the sound source locations may comprise two sub-steps of demining co-ordinate locations in the 3D space 22 for generating the sources 24 of the sound stimuli (based on the initial positioning information of the subject), and subsequently issuing the control instructions to the acoustic apparatus 12 to cause generation of the defined sound sources at the determined locations.

Determining the locations may make use of an algorithm which receives as input the subject position information from the position sensing apparatus 14, and the desired sound source location information from the datastore 40 (defined relative to position or pose of features of the subject's head), and outputs a set of position co-ordinates, relative to the co-ordinate system 26 of the 3D space, at which each of the sound sources should be generated.

After determining the sound source locations, once the test routine starts, the processing arrangement 16 controls the source locations to be generated at the calculated positions in the 3D space by issuing control instructions to the acoustic apparatus 12.

These locations of the sound sources 24 in the 3D space remain fixed relative to a reference frame 26 of the 3D space after the configuration, independent of subsequent movement of the subject's head. Thus, this set-up phase can be understood as a calibration phase, in which the sound source locations are calibrated according to an initial position of the subject's head and/or head features.

Optionally, adjustment or alterations may be made to the sound stimuli source locations retrieved from the test protocol datastore 40 for the given test protocol, based on detected subject head or ear geometry. These factors are known to alter the perceived location and other properties of sound through diffraction effects. The adjustments may be performed by using an appropriately trained AI model, and based on acquired position sensing data from the position sensing apparatus 14. This algorithm may be trained to be applied to the generic sound source locations as stored in the dataset, or to the converted set of source locations defined relative to the co-ordinate system of the 3D space.

Once the properties of the sound distribution which is to be generated in the test routine have been established, the test routine can begin. Optionally, the processing arrangement 16 may include a dedicated test execution module 162 for performing the functional steps of the test routine.

The processing arrangement 22 may begin the test routine by transmitting the determined sound stimulus properties and 3D source locations to the acoustic apparatus 12, or instructions may otherwise be transmitted to the acoustic apparatus to cause the apparatus to generate the required one or more sound stimuli at the required sound source locations in the 3D space 22.

By way of example, the acoustic apparatus 12 may include a local controller part and a sound generation part. The local controller may configured to map the received instructions or information to the necessary control parameters or settings for controlling the acoustic generator part to generate the sound stimuli. For example, in the case of a laser-based sound generator, these settings may include laser intensity, scan or pulse frequency, laser light frequency and emission angle or direction.

Once, the sound configuration with defined sound stimuli 24 is generated, the subject instructions are generated and a sensory output 20 device is controlled to generate a sensory representation of the instructions for the subject.

As noted above, the instructions 43 to be generated may optionally be stored on the test routine datastore 40. Alternatively, for each test routine or protocol, a separate datastore may store the corresponding instructions to be issued to the subject. This may be based on acquiring a unique test routine ID or identifier from the datastore storing the set-up configurations for instance, or the test ID may be provided by the user at the user interface 52. This can be used to query a separate instruction datastore, e.g. storing a look-up table, to obtain the necessary subject instructions.

In general, the instructions are for prompting the subject to move their head to a location at which the perceived sound matches a defined (target) acoustic quality.

Once the instructions have been generated, a position or pose in the 3D space of at least one or more anatomical features of the subject's head after generation of the instructions, is determined, based on acquiring data from the position sensing apparatus 14. As mentioned above, a record of which particular position or pose measurements 44 should be acquired for each test routine may be stored in the test routine datastore 40. Instructions may be issued to the position sensing apparatus to acquire the defined positioning information. The one or more algorithms subsequently applied to derive the hearing property measures may then be configured to receive these specified position measurements as inputs.

Once the position sensing data has been acquired, an analysis procedure is applied to determine one or more quantitative properties of the subject's sense of hearing based on the positioning information. The processing arrangement may optionally include a dedicated analysis module 163 for performing this task. This may be done by applying one or more algorithms, equations or functions. In some examples, relative position information between at least one of the sound stimulus source locations and one or more features of a subject's head (or a mid-point between two features) may be provided as input information to the algorithm. The test routine datastore 40 may store for each different test routine a record of the one or more algorithms 45 to be applied for deriving the hearing properties based on the acquired position data.

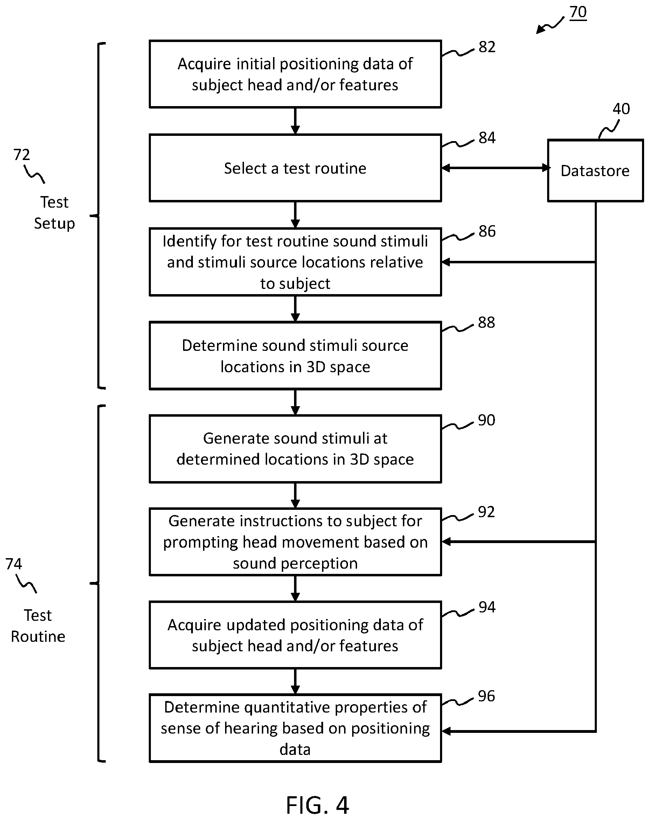

By way of summary of the above, a full example workflow 70 of the system 10 according to one or more embodiments will now be briefly outlined, with reference to Fig. 4.

The workflow 70 for implementing a hearing test can be divided into two phases: a test set-up phase or protocol 72, and a test routine execution phase or protocol 74. The test set-up phase comprises acquiring 82 initial positioning information of the subject's head and/or one or more anatomical features thereof. This can include sub-steps of acquiring spatial sensing data and applying image processing algorithms to determine a position or pose of the one or more subject features. A test routine which is to be performed is selected 84, for example responsive to a user control input from a user interface 52, and/or using a test selection algorithm adapted to automatically select a test. The test may be selected from a store of template test protocols or routines stored in a test routine datastore 40. The particular sound distribution which must be generated for the selected test is identified 86. For example this is retrieved from the datastore 40. The sound distribution includes a set of sound stimuli to be generated having defined acoustic properties and defined locations relative to the subject. For example the datastore includes a lookup table which can be queried for the sound stimuli information for the given test identity. The required source locations for the sound stimuli within the co-ordinate system 26 of the 3D space 22 are then determined 88. For example the generic sound source locations from the datastore (defined relative to subject positioning) may be converted to a set of locations defined in the co-ordinate system 26 of the 3D space 22 based on the initial position information 82 of the subject. The acoustic apparatus is controlled to generate 90 the sound distribution according to the determined sound distribution information. The instructions for the subject are generated 92 and communicated to the subject. Subsequently, updated positioning data of the subject is acquired 94. The position of the subject's head, or one or more features thereof, subsequent to the issuing of the instructions, is then used as in input to one or more algorithms for determining 96 quantitative properties of the subject's sense of hearing.

Example processes for determining quantitative properties of the subject's hearing based on the acquired position or pose data will now be discussed in more detail.

In accordance with at least one set of embodiments, properties of hearing may be determined based on a relative position between one or more features of a subject's head after following the instructions (e.g. ear canal entrances) and the location in the 3D space of at least one of the generated sound stimuli.

For example, in general, the instructions are for prompting the subject to move their head to a location at which the perceived sound matches a defined acoustic quality. Properties of the subject's hearing can then be discerned based on the position of the subject's head or ears at which they perceive that acoustic property.

For example, the instructions may prompt the subject to move their head such that one or more of the sound sources is perceived to be at a particular target location relative to them (e.g. directly above their head, and/or at a mid-point between their ears), or to a location at which they experience a maximum or minimum of a particular acoustic property of the sound, e.g. volume or frequency, or at which they experience a particular balance in a property of the sound between two or more sources, e.g. balanced volume or frequency.

The instructions may consist of one step or multiple steps. In the case of multiple-step tests, each subsequent test step may be initiated after a defined time period, or when the subject's movement has sensed to have ceased for a pre-defined period, indicating that they have completed the instructions.

Once the subject has followed the instructions, the determining the quantitative properties of the subject's hearing may comprise calculating, based on the data from the position sensing apparatus: a displacement of at least one or more anatomical features of the subject's head, or a mid-point between two features (such as the ears), from a source location in the 3D space of at least one of the sound stimuli.

This information may then be used as part of a subsequent calculation of a property of the subject's hearing, e.g. through a function or equation, a look-up table, or a machine learning algorithm.

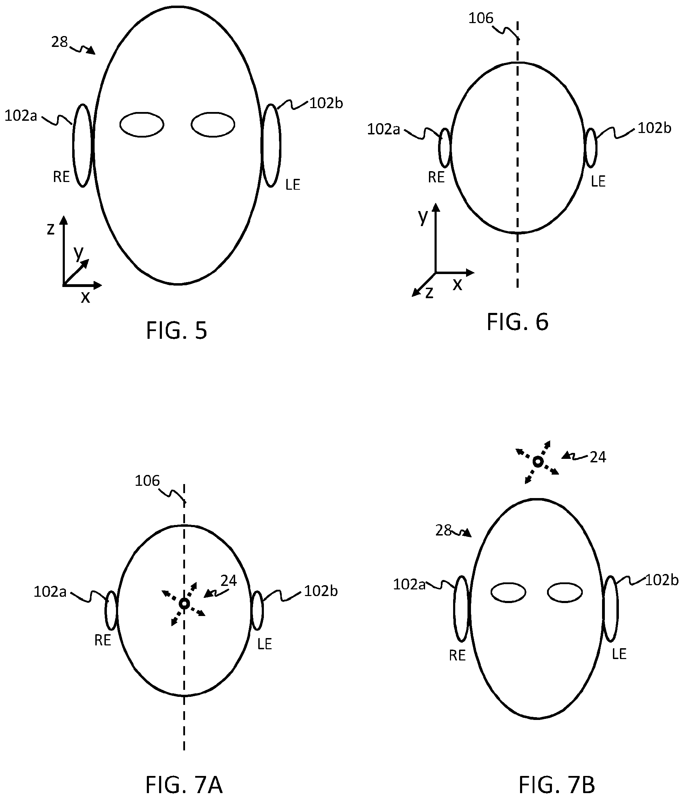

One example test routine is for measuring left/right ear sensitivity imbalance. It may optionally also measure ear sensitivity magnitude. An example of this test will now be outlined, with reference to Figs. 5-8.

Fig. 5 schematically depicts the subject's head 28 from a front view. Fig. 6 depicts the subject's head from an aerial view (top-down), where the subject is facing in the direction of the bottom of the page. As part of this test routine, a location of the subject's right ear (RE) 102a, and left ear (LE) 102b in the 3D space will be detected using the positioning data of the position sensing apparatus 14. For example, a position of an entrance to the ear canal of each of the right ear 102a and left ear 102b may be determined. Other points on the ear may alternatively be used as the measurement point. From the measurements of the ear locations, a location of a midpoint or mid-line horizontally equidistant between the locations of the ears will be determined by the position sensing apparatus 14 or by the processing arrangement 16. The horizontal direction in this context is defined by the direction of a virtual straight line directly connecting the locations of the ears (the x-direction according to the illustrated co-ordinate axes, which axes correspond to a reference frame of the subject's head). Thus, there may be detected a mid-point along such a virtual straight line directly connecting the ear locations, or there may be detected an axis, characterized by a direction vector, which orthogonally bisects this straight line. The latter option is illustrated schematically in Fig. 6 which shows a detected mid-point axis 106 between the subject's ears, which intersects a virtual straight line between the ear locations at a mid-point.

During execution of the test, a sound distribution is generated comprising a single sound stimulus having a source location 24 which is controlled to be at a point directly above the subject's head, and horizontally (x-axis) aligned with the horizontal mid-point or mid-line axis 106 between the subject's ears. This is illustrated in Fig. 7A and 7B. By way of non-limiting illustration, a single sound source might be generated at a distance of 6-10 cm above the subject's head, for example approximately 8 cm above their head.

The subject is then issued instructions to move their head along the horizontal direction (defined as the direction of the axis extending directly between their ears), i.e. left or right, until they perceive the sound source 24 to be at a location horizontally equidistant between their right 102a and left 102b ear, e.g. directly above their head. Another way of issuing these instructions is to ask the subject to move their head horizontally until they perceive the sound source to be of equal loudness in both ears (i.e. the loudness in balanced between their ears).

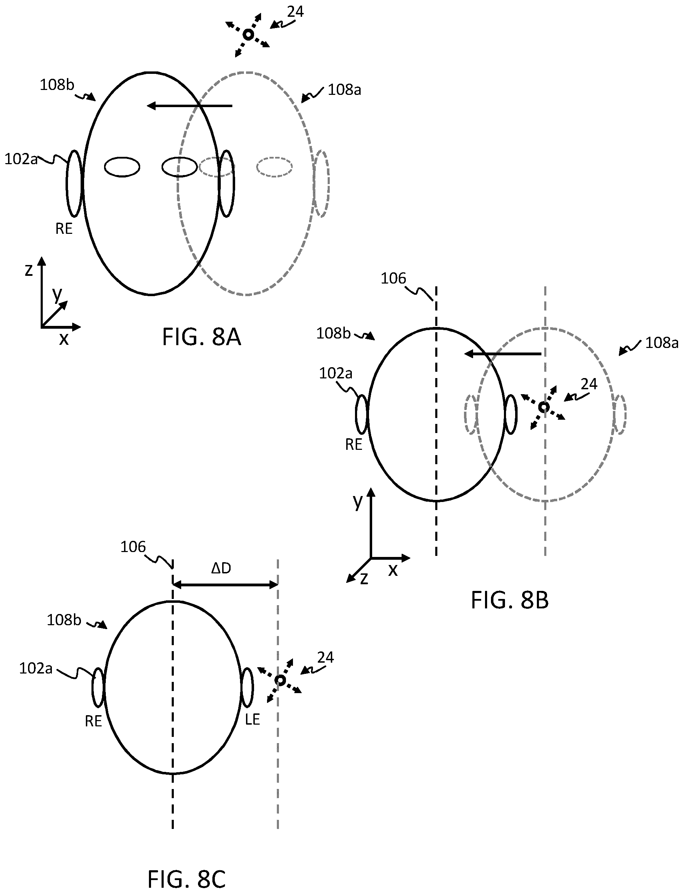

This is illustrated in Figs. 8A and 8B, which show the subject moving their head from an initial position 108a (as measured by the position sensing apparatus 14 before issue of the instructions, in a set-up phase 72) to an adjusted position 108b in which they perceive the specified sound quality or property. The subject has shifted their head to their right. The sound stimulus source location 24 remains fixed relative to a reference frame of the 3D space independent of the movements of the subject's head. After the movement, the new position 108 of the subject's ears 102a, 102b is measured by the position sensing apparatus 14. A new position of the mid-point or mid-line axis 106 between the subject's ear locations is detected. A deviation or displacement, ΔD, in the horizontal direction (meaning a direction defined by a straight axis between the subject's ears) of this mid-point or mid-line axis 106, from the location of the sound stimulus source 24 is determined by the processing arrangement 22. The location of the sound stimulus source 24 is known from the set-up phase 72 of the test. In some examples, this deviation may be calculated as a proportion of the distance between the subject's ears, such that the output may be given as a measure of the hearing imbalance between the ears, e.g. 5% lower sensitivity in left ear. The calculated deviation, either in absolute or proportional terms may be directly used as the quantitative measure of the subject's left/right ear insensitivity, or a further algorithm may be applied which converts this measure to a different metric representative of this hearing property.

It is noted that Fig. 8, for ease of illustration, somewhat exaggerates the magnitude of subject head movement that might be expected during the test. In practice, smaller horizontal head movements would be typical.

In a variation on the above test protocol, the sound stimulus may be initially generated at a source location 24 which is somewhat offset from the mid-point between the subject's ears and the same instructions are issued. The same computation process may be followed as described above, wherein a final displacement, ΔD, between source location and ear mid-point is determined. For instance, and by way of non-limiting example, the sound source may be generated at a distance approximately 2-4 cm horizontally offset from the mid-point between the ears.

Additionally or alternatively, in a further variation on this test routine, instead of generating a single sound stimulus directly above the head of the subject, two sound stimuli may be generated with source locations 24 either side of the subject's ears. For example, and by way of non-limiting illustration, two points a distance approximately 8-10 cm horizontally away from each ear may be generated. Each sound source may be aligned on the horizontal axis connecting the patient's ears. The subject is asked to move their head to a point where they perceive the loudness of the two sound sources as equal, or where they perceive their head to be equidistant between the two sources.

A horizontal displacement may then be measured between the midpoint between the subject's ears (or a mid-line axis 106 between the ears) and a midpoint between the two sound stimuli (i.e. a mid-point of a horizontal straight line connecting the horizontal locations of the stimuli). The horizontal direction is defined as specified above (i.e. relative to the subject's head). This variation will also provide a measure of left-right ear sensitivity imbalance. In this disclosure, a 'midpoint' between two points means a midpoint along a straight line directly connecting those two points.

The above represents just one example test routine, and further routines for measuring other properties are also possible.

For example, a directional sensitivity of the subject's ears can be measured based on instructions which prompt the subject to point their head in the direction of the perceived source location of a sound stimulus. Subsequently, the position sensing apparatus may be used to measure a pose of the subject's head in the 3D space. An angular displacement between the a first directional vector defining the facing direction of their head, and a second directional vector, starting from the same origin as the first, and pointing toward the sound stimulus source location, may provide a measure of directional sensitivity error. The first directional vector may be a direction along a mid-line axis 106 as defined in the example discussed above and shown in Figs. 5-8.

Directional sensitivity can decline in old age, and thus is a valuable property of the hearing to be able to measure accurately.

This test can be performed with a single sound stimulus with a single source location or with a plurality of sound stimuli at different source locations. The latter case can be used to provide a measure of sensitivity of hearing function to concurrent noise. This is sometimes referred to as the 'cocktail party problem', namely the ability to selectively attend to a single source of sound among a background of multiple different sound sources. Thus, in one example test routine, a plurality of sound stimulus sources are generated at different locations, and the subject is asked to turn their head in the direction of a sound source having a particular acoustic quality (defined in the instructions), e.g. a sound source which appears to be saying a particular word.

A further example test routine may be for measuring subject frequency sensitivity. By way of example, a regular spatial array (e.g. grid), or other defined spatial arrangement, of multiple sound sources is generated in the 3D space with varying acoustic frequencies. The instructions ask the subject to 'look at' (angle their head towards) a point where the sound is perceived to be the loudest. The summation of the sound and perceived amplitudes will cause the perceived sound direction to vary according to the subject's amplitude sensitivity profile at different frequencies. There may be a pre-defined 'normal' or reference sound perception direction which represents a normal frequency sensitivity. A deviation (e.g. angular deviation) between this normal direction, and the direction in which the subject is facing after following the instructions may be determined. This may provide the quantitative measure of the property of frequency sensitivity error.

Further example test routines may determine directional sensitivity in planes other than the horizontal (x-axis). These may follow a process identical to that descried above in relation to Figs. 5-8, but wherein, the subject is instructed to
move their head forward or backward (i.e. in the forward/back plane, or y-direction) until they perceive the sound as being directly above their head, i.e. lying on a straight line directly extending between their ears. A deviation (in the forward-backward direction) of a horizontal mid-point between the subject's ears and the actual location of the sound source may be measured as a metric of directional sensitivity imbalance.

Raise/lower their head until the sound is perceived to be at the same vertical level as the subject's ear. A vertical deviation between the line connecting the subject's ears after following the instructions and an actual position of the sound source may be measured and used as a metric of directional sensitivity imbalance.

In accordance with one or more embodiments, as an additional feature, the acoustic apparatus may generate distractor sounds, which can test the subject's ability to distinguish attended audio from controlled noise which has controlled directional properties. These distractor sounds may be generated for example during a directional sensitivity test, and the sensitivity test run once without the sounds and once with the sounds, and a comparison between the results used as a metric of ability to selectively directionally attend to sound sources.

Embodiments of the invention outlined in the present disclosure provide a system operable to collect objective measurements of a subject's hearing ability, which removes error in the delivery of the test stimulus and the method of subject reporting. Specifically, at least the following advantages are enabled:
ability to perform a diagnostic hearing test in a fully automated way;
quantitative and objective measurement of spatial hearing inaccuracies;
very accurate assessment through precise placement of real sound sources;
ability to assess the patient's real-world directional hearing ability, including the acoustic transfer function of their head.

Embodiments of the invention described above employ a processing arrangement. The processing arrangement may in general comprise a single processor or a plurality of processors. It may be located in a single containing device, structure or unit, or it may be distributed between a plurality of different devices, structures or units. Reference therefore to the processing arrangement being adapted or configured to perform a particular step or task may correspond to that step or task being performed by any one or more of a plurality of processing components, either alone or in combination. The skilled person will understand how such a distributed processing arrangement can be implemented.

The one or more processors of the processing arrangement can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processing arrangement or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A system (10) for use in assessing hearing of a subject (8), the system comprising:
an acoustic apparatus (12) operable to generate a sound distribution in a 3D space (22), the sound distribution comprising one or more sound stimuli having controllable real or virtual sound source locations (24) in the 3D space;
a position sensing apparatus (14) for use in detecting a position or pose of at least one or more anatomical features of a subject's head (28) in the 3D space during a testing routine;
a processing arrangement (16) configured to perform a testing routine (74), the testing routine comprising:
generating a sound distribution in a 3D space (22) comprising one or more sound stimuli having defined sound source locations (24) in the 3D space, using the acoustic apparatus (12);
generating instructions for communication to the subject during generation of the sound distribution, the instructions for prompting the subject to respond to the sound stimuli with movements of their head (28) based on their perception of the sound;
determining a position or pose in the 3D space (22) of at least one or more anatomical features (102a, 102b) of the subject's head after generation of the instructions, based on data from the position sensing apparatus (14); and
determining one or more quantitative properties of the subject's sense of hearing based on a position or pose of the at least one or more anatomical features of the subject's head in the 3D space after issuance of the instructions.

2. A system (10) as claimed in claim 1, wherein the instructions are for prompting the subject (8) to move their head to a location at which the perceived sound matches a defined acoustic quality.

3. A system (10) as claimed in claim 1 or 2, wherein the processing arrangement (16) is further adapted to, in advance of generating the instructions,
determine (82) an initial position or pose (108a) of at least one or more anatomical features of the subject's head (28) in the 3D space (22); and
configure the sound stimuli source locations (24) in the 3D space (22) based on said initial position or pose of the at least one or more anatomical features of the subject's head.

4. A system (10) as claimed in claim 3, wherein the configuring the sound stimuli source locations (24) comprises:
controlling the source locations (24) to be at defined positions relative to the determined initial position(s) (108a) of the at least one or more anatomical features of the subject's head, and
wherein the locations of the sound sources in the 3D space (22) remain fixed relative to a reference frame of the 3D space after the configuration, independent of subsequent movement of the subject's head.

5. A system (10) as claimed in claim 3 or 4, wherein the system further comprises a datastore (40) storing configuration instructions for each of a set of different testing routines, each testing routine for assessing different sets of one or more properties of the subject's sense of hearing, wherein the configuration instructions for each testing routine define at least:
a set (41) of one or more sound stimuli to be generated; and
spatial locations (42) relative to at least one or more anatomical features of the subject's head, or relative to a midpoint between two or more anatomical features, at which the real or virtual sources (24) of the sounds should be generated.

6. A system (10) as claimed in any of claims 1-5, wherein the position sensing apparatus (14) comprises: at least one imaging device; and/or a LIDAR sensing apparatus.

7. A system (10) as claimed in any of claims 1-6, wherein the determining a position in 3D space (22) of at least one or more anatomical features of the subject's head comprises determining a position in 3D space of at least one of the subject's ears (102a, 102b).

8. A system (10) as claimed in any of claims 1-7, wherein the acoustic apparatus (12) comprises:
a laser-based sound generation apparatus;
an ultrasound apparatus; and/or
one or more speakers.

9. A system (10) as claimed in any of claims 1-8, wherein the determining the quantitative properties of the subject's hearing comprises determining one or more of:
a left/right ear sensitivity imbalance;
an ear sensitivity magnitude;
an ear directional sensitivity.

10. A system (10) as claimed in any of claims 1-9, wherein the determining the quantitative properties of the subject's hearing comprises calculating, based on the data from the position sensing apparatus (14):
a displacement (ΔD) of at least one or more anatomical features of the subject's head, or a midpoint therebetween, from a source location in the 3D space (22) of at least one of the sound stimuli (24), and/or
a pose of the subject's head in the 3D space.

11. A system (10) as claimed in any of claims 1-10, wherein the system further comprises a sensory output device (20) for generating a sensory output indicative of the generated instructions.

12. A computer-implemented method (70) for testing hearing of a subject, the method comprising performing a testing routine (72) comprising:
generating (90) a sound distribution in a 3D space (22), the sound distribution comprising one or more sound stimuli having controllable real or virtual sound source locations (24) in the 3D space;
generating (92) instructions for communication to the subject during generation of the sound distribution, the instructions for prompting the subject to respond to the sound stimuli by performing movements of their head based on their perception of the sound;
determining (94) a position in the 3D space of at least one or more anatomical features of the subject's head after generation of the instructions; and
determining one or more quantitative properties of the subject's sense of hearing based on a position or pose of the at least one or more anatomical features of the subject's head in the 3D space after issuance of the instructions.

13. A method (70) as claimed in claim 12, wherein the instructions are for prompting the subject to move their head to a location at which the perceived sound exhibits a defined target acoustic property.

14. A method as claimed in claim 12 or 13, further comprising, in advance of generating the instructions:
determining (82) an initial position or pose (108a) of at least one or more anatomical features of the subject's head in the 3D space; and
configuring (86, 88) the sound stimuli source locations based on said initial position or pose of the at least one or more anatomical features of the subject's head.

15. A computer program product comprising computer program code, the computer program code being executable on a processing arrangement, wherein,
when the processing arrangement is operatively coupled with an acoustic apparatus operable to generate a configurable sound distribution in space, and with a position sensing apparatus,
the code is configured to cause the processing arrangement to perform a method in accordance with any of claims 12-14.
